Europäisches Patentamt

European Patent Office

Office européen des brevets

(19)

(11) Publication number: **0 340 320**

**A1**

(12) ## EUROPEAN PATENT APPLICATION

(21) Application number: **88107001.5**

(22) Date of filing: **02.05.88**

(51) Int. Cl.⁴: **A61F 5/01**

(43) Date of publication of application:
**08.11.89 Bulletin 89/45**

(84) Designated Contracting States:
**AT BE CH DE ES FR GB GR IT LI LU NL SE**

(71) Applicant: **INTEC Advanced Computerized
Systems Medical Ltd.
Hillel Street, 5
Jerusalem(IL)**

(72) Inventor: **Livni, Michael, Dr.
6/1 Hazav Street
Mevasseret(IL)**

(74) Representative: **Dreiss, Hosenthien &
Fuhlendorf
Gerokstrasse 6
D-7000 Stuttgart 1(DE)**

(54) Inflatable cushion affixable to a diaper.

(57) There is disclosed an inflatable cushion adapted to be affixed to a diaper or babies panties. It comprises a closable inflating opening (2,3) and moans (9) for affixing permanently or releasably the same to said diaper or panties, in the set-up position thereof, at the groin, between the thighs of the infant and supporting them in an abducted position.

FIGURE 1a

FIGURE 1b

EP 0 340 320 A1

## Inflatable cushion affixable to a diaper

The present invention is directed to an inflatable cushion, made from a non-water absorbent material adpated to be affixed to a diaper suitable for the preventive treatment of a congenital defect, congenital dislocation or dysplasia of the hip joint also in cases not diagnosed as such, immediately or shortly after birth. There is some confusion in the use of these terms, since dysplasia means only a dislocatable hip joint, and a distinction is important when considering the incidence of the defect. On the average, according to some authorities, the incidence of dysplasia seems to be about one percent of live births, but the fully developed cases of dislocated hips are considerably less frequent, also if now treated. This leads some authorities to the conclusion that there is a possibility of spontaneous stabilization of dysplastic hips in early infancy.

In any case, where there is only mild dysplasia it might not always be diagnosed even by trained personnel, especially during earliest infancy. Therefore, it is possible that a number of cases may go undetected until the child is two or three months old, when X-ray examination will provide conclusive proof of whether there is a dysplasia/ dislocation or not. The presence of such abnormalities calls for treatment by immobilization with appropriate splints, usually keeping the legs abducted at approximately 60° and appropriately flexed. This posture which allows the head of the femur to exert pressure on the acetabulum (socket) has been proven to enhance good development as well as stabilization of the hip joint.

An adducted position of the thighs in infancy may lead to a higher incidence of dysplastic, respectively dislocated hips. This is supported by observations of such a higher incidence in populations where it is customary to swaddle infants with thighs in an adducted position. The importance of holding the thighs in an abducted position is now well recognised, and the present widespread practice is to use a makeshift arrangement, i.e., to use two or perhaps more layers of diapers, providing some semi-rigidity and also a kind of wad in region of the groin to achieve abduction. However, this practice is only partially effective as the material of the diapers is water absorbent and loses all rigidity and resistance on becoming wet. Furthermore, it involves additional work, expense, and inconvenience.

As to the splints used in the therapeutical treatment of diagnosed cases (not forming part of the present invention), these are in most cases very rigid splints. However, the use of foamed plastic cushions, trapped to the thighs and permitting certain freedom of adductive movements but capable of recovering their shape, as also been recommended in the literature.

In view of the fact that the present invention inflatable cushion serving as means for holding the thighs of the infant in the abducted position is non-water absorbent, the problem of becoming ineffective in the wet state does not arise.

Our co-pending European Patent Application No. 87 104 287.1 discloses a diaper for infants characterised in that it comprises means integral with the same or appropriately insertable into the same, said means being non-water absorbent and being located in the set-up position of the diaper, at the groin, between the thighs and supporting them in an abducted position. The advantages of this diaper are demonstrated and discussed at large in our said European patent application, incorporated herein by reference.

The present invention provides for a particular abduction means, namely an inflatable cushion made from a non-water absorbent material, adapted to be affixed permanently or releasably to a diaper or babies' waterproof panties.

More particularly the invention provides for an inflatable cushion, made from non-water absorbent material, having a closable inflating opening, provided with means for permanently or releasably affixing the same to a diaper or babies' waterproof panties, at the groin, between the thighs of the infant, and supporting them in an abducted position. The inflatable cushion of the present invention may be made by air-tightly heat sealing two layers of suitable plastic material leaving a suitable free opening for inflating. The cushion may then be inflated by inserting a tube containing a self-sealing valve in said opening. Alternatively, the inflating tube may be formed as an integral part of the cushion, as will be described hereafter in more detail. The inflating may be done by mouth or by a small aerosol containing a suitable gas. It is also possible to inflate the cushion by a gas-producing mixture of chemicals or by special containers of suitable compressed gas. The cushion may be completely, or only partially inflated, to achieve the desired extent of abduction of the thighs. The cushion is provided with means for permanently or releasably affixing the same to a diaper or babies' waterproof panties, in the set-up position thereof, at the groin, between the infant's thighs, and holding the same in an abducted position.

A preferred embodiment of a cushion according to the present invention will now be described in detail, on hand of the accompanying drawings, in which:

Figure 1a is a top view of a cushion of the present invention;

Figure 1b is a cross-sectional view of the cushion of Fig. 1a, in the inflated state, along the line A-A of Fig. 1a;

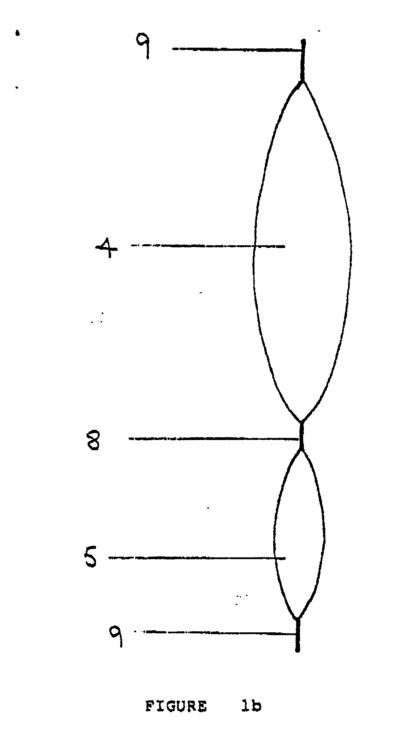

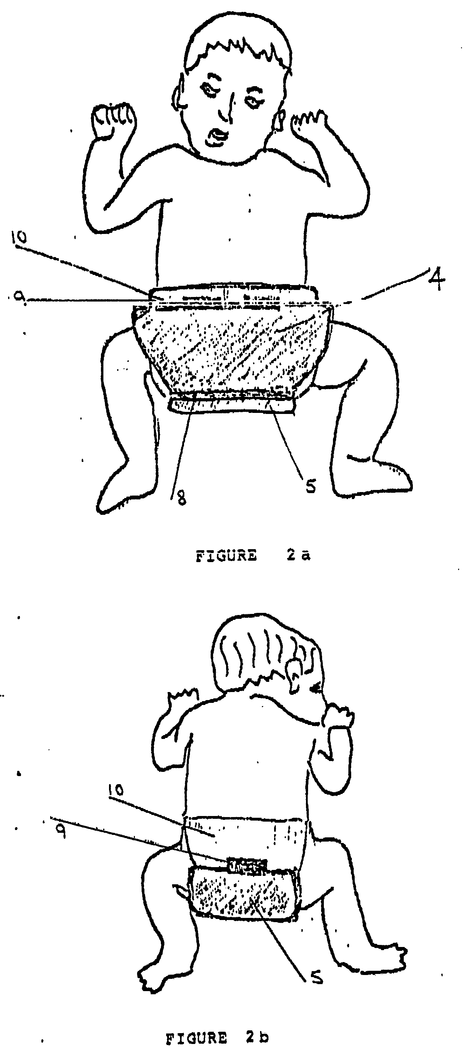

Fig. 2a is a schematic front view of the cushion of Figs. 1a and 1b, affixed to a diaper, in the set-up position:

Figure 2b is a rear view of the illustration of Fig. a; and

Figure 3 is a schematic front view of the cushion of the present invention affixed to waterproof panties.

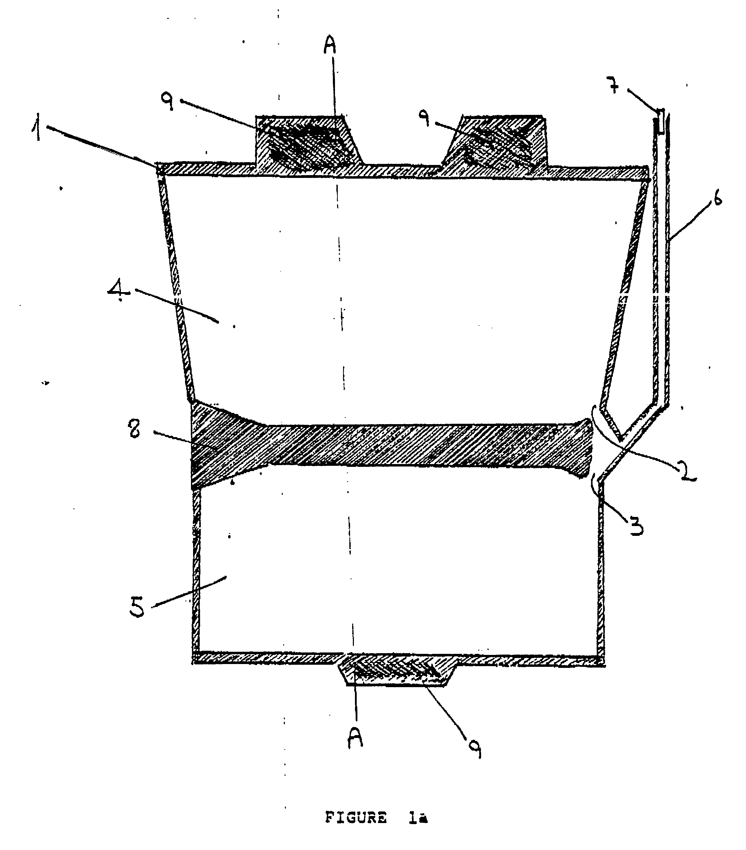

Figure 1a shows a preferred embodiment of an inflatable cushion according to the present invention from a top view. The cushion 1 is made from two layers of suitable plastic material, heat sealed along practically the whole circumference thereof, except for openings 2 and 3, serving as air inlets. Front part 4 is separated from rear part 5 by a heat-sealed strap 8, in the region corresponding to the groin, which serves as a folding axis.

In the illustrated embodiment, an inflating pipe 6 is integrally formed with the cushion. Inflating may be by mouth, with aid of tube 7, inserted into pipe 6, or automatic, as referred to above. The cushion is provided with means 9 for permanently or releasably affixing the same to a diaper or waterproof-panties, preferably suitable adhesive straps.

Figure 1b illustrates the cushion of Fig. 1a, in a cross-sectional view, in the inflated state. The cushion may be multiple-use, particularly when intended to be releasably affixed, or disposable, particularly when intended to be permanently affixed. Naturally, the affixing means 9 have to be suitable for either permanent or releasable affixing. Figures 6a and 6b illustrate a cushion according to Figures 1, affixed to a diaper 10, in the set-up position. The thighs abduction angle empirically adjusted by completely or only partially inflating the cushion.

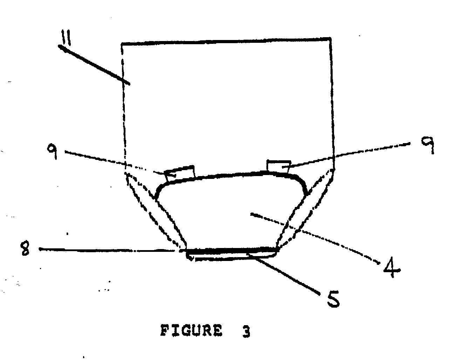

Figure 3 schematically illustrates a cushion according to the invention, affixed to babies' panties 11. While only few specific embodiments have been described in detail, the invention is not limited thereto and is only defined by the scope of the appended claims.

## Claims

1. An inflatable cushion adapted to be affixed to a diaper or babies panties, characterized in that it comprises a closable inflating opening and means for affixing permanently or releasably the same to said diaper or panties, in the set-up position thereof, at the groin, between the thighs of the infant and supporting them in an abducted position.

2. An inflatable cushion according to claim 1, made from two sheets of suitable plastic material, heat-sealed together, leaving a closable opening for inflating the same.

3. An inflatable cushion according to claim 2 characterized in that it further comprises an inflating pipe, integral with the same.

4. An inflatable cushion according to any of the preceding claims characterized in that it further comprises a heat-sealed strap, at the region corresponding to the groin.

5. An inflatable cushion according to any one of the preceding claims wherein said affixing means are suitable adhesive straps.

6. An inflatable cushion according to any one of the preceding claims with reference to and as illustrated in the accompanying drawings.

7. Baby diaper equipped with an inflatable cushion according to any one of the preceding claims.

8. A diaper according to claim 7 wherein the said cushion is permanently affixed thereto.

9. Babies' panties equipped with an inflatable cushion according to any one of the preceding claims.

10. Babies' panties according to claim 9 wherein the said cushion is permanently affixed thereto.

FIGURE 1a

FIGURE 1b

FIGURE 2 a

FIGURE 2 b

FIGURE 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE-U-1 984 102 (PLAAT) <br> * claims 1-4; figures 1,2 * | 1 | A 61 F 5/01 |
| A | | 2-4 | |
| A | EP-A-0 050 050 (LAMBERT) <br> * claims 1,20; figures 1-8 * | 1,7-10 | |
| A | DE-U-6 909 273 (POPP) <br> * claims 1,4; figure 1 * | 1,9 | |
| A | US-A-3 823 712 (MOREL) <br> * column 1, lines 9-19; claim 1 * | 1 | |
| A | GB-A-2 178 663 (AMBROPLASTICS LTD.) <br> * claims 1,2 * | 1-3 | |

| | TECHNICAL FIELDS SEARCHED (Int. Cl.4) |
|---|---|
| | A 41 B 13/00 <br> A 61 F 5/00 |

The present search report has been drawn up for all claims

| Place of search | Date of completion of the search | Examiner |
|---|---|---|
| BERLIN | 05-12-1988 | KANAL P K |

EPO FORM 1503 03.82 (P0401)